Europäisches Patentamt
European Patent Office
Office européen des brevets

(19) (11) Veröffentlichungsnummer: **0 236 897**
**A1**

(12) **EUROPÄISCHE PATENTANMELDUNG**

(21) Anmeldenummer: 87102912.0

(22) Anmeldetag: 02.03.87

(51) Int. Cl.³: C 07 D 213/78
A 01 N 43/40, A 01 N 47/06

(30) Priorität: 13.03.86 DE 3608383

(43) Veröffentlichungstag der Anmeldung:
16.09.87 Patentblatt 87/38

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Fest, Christa, Dr.
Im Johannistal 20
D-5600 Wuppertal 1(DE)

(72) Erfinder: Brandes, Wilhelm, Dr.
Eichendorffstrasse 3
D-5653 Leichlingen(DE)

(72) Erfinder: Hänssler, Gerd, Dr.
Am Arenzberg 58a
D-5090 Leverkusen 3(DE)

(72) Erfinder: Reinecke, Paul, Dr.
Steinstrasse 8
D-5090 Leverkusen 3(DE)

(54) **Arylsulfonyl-pyridinaldoxim-Derivate.**

(57) Arylsulfonyl-pyridinaldoxim-Derivate der Formel (I)

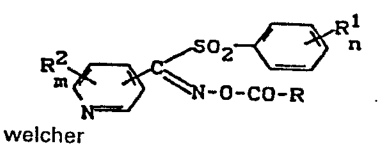

(I)

in welcher
R, $R^1$, $R^2$, n und m die in der Beschreibung gegebenen Bedeutungen haben sowie ihre Verwendung als Schädlingsbekämpfungsmittel.

Die neuen Arylsulfonyl-pyridinaldoxim-Derivate der Formel (I) können aus geeigneten Arylsulfonyl-pyridinaldoximen und geeigneten Carbonylverbindungen hergestellt werden.

EP 0 236 897 A1

0236897

BAYER AKTIENGESELLSCHAFT
Konzernverwaltung RP
Patentabteilung

5090 Leverkusen, Bayerwerk
Bas/Ke-c
Ia

## Arylsulfonyl-pyridinaldoxim-Derivate

Die vorliegende Erfindung betrifft neue Arylsulfonyl-pyridinaldoxim-Derivate, ein Verfahren zu ihrer Herstellung und ihre Verwendung als Schädlingsbekämpfungsmittel, insbesondere als Fungizide.

Es sind bereits eine Reihe von Aldoxim-Derivaten bekannt. So z.B. Arylsulfonylbenzaldoxime, wie das α-Phenylsulfonyl-2,6-dichlor-benzaldoxim, und ihre Verwendung als Schädlingsbekämpfungsmittel, vor allem ihre Verwendung in Mitteln zur Bekämpfung von Weizensteinbrand, ist bekannt (vgl. Schweizer Patent Nr. 423.350). Weiterhin sind Phenyl-pyridinaldoxime, wie z.B. das Phenyl-O-ethylcarbonyl-pyridinaldoxim, und ihre Antihistamin-Wirkung bekannt (vgl. J. pharm. Sci. 56(1967) Nr. 10, S. 1354-1357).

Es wurden neue Arylsulfonyl-pyridinaldoxim-Derivate der allgemeinen Formel (I)

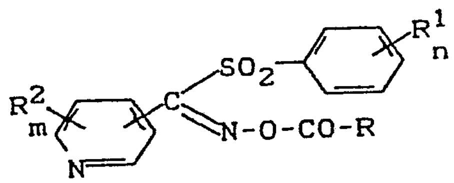

(I)

gefunden, in welcher

R für Alkyl oder Alkoxy steht,

$R^1$ für Halogen, Alkyl, Alkoxy, Alkylthio, Halogenalkyl, Halogenalkoxy, Halogenalkylthio, Nitro, Alkoxycarbonyl oder Alkylcarbonylamino steht,

$R^2$ für Halogen oder Alkyl steht,

n für eine ganze Zahl 0, 1, 2, 3, 4 oder 5 und

m für eine ganze Zahl 0, 1, 2, 3 oder 4 stehen,

wobei die Substituenten in den Ringen gleich oder verschieden sein können.

Weiterhin wurde gefunden, daß man die Arylsulfonyl-pyridinaldoxim-Derivate der allgemeinen Formel (I)

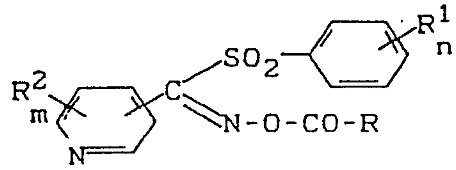

(I)

in welcher

R für Alkyl oder Alkoxy steht,

$R^1$ für Halogen, Alkyl, Alkoxy, Alkylthio, Halogenalkyl, Halogenalkoxy, Halogenalkylthio, Nitro, Alkoxycarbonyl oder Alkylcarbonylamino steht,

$R^2$ für Halogen oder Alkyl steht,

n für eine ganze Zahl 0, 1, 2, 3, 4 oder 5 und

m für eine ganze Zahl 0, 1, 2, 3 oder 4 stehen,

wobei die Substituenten in den Ringen gleich oder verschieden sein können, erhält, wenn man Arylsulfonylpyridinaldoxime der allgemeinen Formel (II)

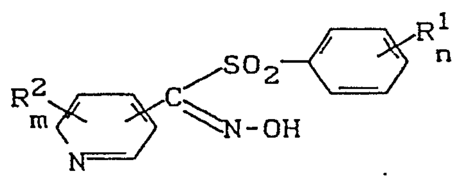

(II)

in welcher

$R^1$, $R^2$, n und m die oben angegebenen Bedeutungen haben, mit Carbonylverbindungen der allgemeinen Formel (III)

$$X - CO - R \qquad (III)$$

in welcher

R die oben angegebenen Bedeutungen hat und

X für ein Halogenatom, vorzugsweise Chlor, oder für den Rest -O-COR steht,

gegebenenfalls in Gegenwart eines Lösungs- oder Verdünnungsmittels und gegebenenfalls in Gegenwart eines Säurebindemittels umsetzt.

Die erfindungsgemäßen Arylsulfonyl-pyridinaldoxim-Derivate der Formel (I) weisen starke biologische, vor allem fungizide Eigenschaften auf.

Überraschenderweise zeigen dabei die erfindungsgemäßen Verbindungen eine erheblich höhere, vor allem fungizide Wirksamkeit als die aus dem Stand der Technik bekannten Verbindungen, die strukturell und/oder wirkungsmäßig sehr naheliegende Verbindungen sind.

Die erfindungsgemäßen Verbindungen der Formel (I) können als syn- oder anti-Isomere oder als deren Gemische in unterschiedlicher Zusammensetzung anfallen. Die Erfindung bezieht sich sowohl auf die reinen Isomeren als auch auf die Isomerengemische.

Die Alkylreste R, $R^1$ und $R^2$ sowie die Alkylteile in den Alkoxy-Resten in R und $R^1$ können geradkettig oder verzweigt sein und enthalten vorzugsweise jeweils 1 bis 6, insbesondere 1 bis 4 Kohlenstoffatome. Beispielhaft seien genannt: Methyl, Ethyl, n-Propyl, iso-Propyl, n-Butyl, sec.-Butyl, iso-Butyl, tert.-Butyl, n-Pentyl, iso-Pentyl, sec.-Pentyl, n-Hexyl, sec.-Hexyl, Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, n-Hexoxy und sec.-Hexoxy.

Die Alkylthio-Reste in $R^1$ können geradkettig oder verzweigt sein und enthalten vorzugsweis 1 bis 6, insbesondere 1 bis 4, besonders bevorzugt 1 bis 3 Kohlenstoffatome. Beispielhaft seien genannt: Methylthio, Ethylthio, n-Propylthio, iso-Propylthio, n-Butylthio, iso-Butylthio, sec.-Butylthio, tert.-Butylthio, n-Pentylthio und n-Hexylthio.

Die Halogenalkylteile in $R^1$ in den Resten Halogenalkyl, Halogenalkoxy und Halogenalkylthio enthalten vorzugsweise jeweils 1 bis 6, insbesondere 1 bis 4, besonders bevorzugt 1 oder 2 Kohlenstoffatome und vorzugsweise 1 bis 9, insbesondere 1 bis 5, besonders bevorzugt 1 bis 4 gleiche oder verschiedene Halogenatome. Beispielhaft seien genannt: Trichlormethyl, Trifluormethyl, Dichlorfluormethyl, Trichlorethyl, Tetrachlorethyl, Trichlormethoxy, Trichlorethoxy, Tetrachlorethoxy, Trichlormethylthio, Trifluormethylthio, Dichlorfluormethylthio, Trichlorethylthio, Tetrachlorethylthio.

Halogen in $R^1$ und $R^2$ auch in den Resten wie Halogenalkyl bedeutet Fluor, Chlor, Brom oder Iod, insbesondere Fluor oder Chlor, wenn an anderer Stelle nicht besonders definiert.

Alkoxycarbonyl und Alkylcarbonylamino in $R^1$ enthält jeweils in den Alkylteilen vorzugsweise 1 bis 4, insbesondere 1 bis 3 Kohlenstoffatome, besonders bevorzugt 1 oder 2 Kohlenstoffatome. Beispielhaft seien genannt: Methoxycarbonyl, Ethoxycarbonyl, n-Propoxycarbonyl, iso-Propoxycarbonyl, n-Butoxycarbonyl, sec.-Butoxycarbonyl, iso-Butoxycarbonyl, tert.-Butoxycarbonyl, Methylcarbonylamino, Ethylcarbonylamino, n-Propylcarbonylamino, iso-Propylcarbonylamino, n-Butylcarbonylamino, iso-Butylcarbonylamino, tert.-Butylcarbonylamino und tert.-Butylcarbonylamino.

n bedeutet vorzugsweise 0, 1, 2, 3 oder 4, insbesondere 0, 1, 2 oder 3,

m bedeutet vorzugsweise 0, 1, 2 oder 3, insbesondere 0 oder 1.

Die erfindungsgemäßen Arylsulfonyl-pyridinaldoxim-Derivate sind durch die Formel (I) allgemein definiert. Bevorzugt sind Verbindungen der Formel (I), in welcher

R für geradkettiges oder verzweigtes Alkyl mit 1 bis 6 Kohlenstoffatomen oder geradkettiges oder verzweigtes Alkoxy mit 1 bis 6 Kohlenstoffatomen steht,

$R^1$ für Halogen, Alkyl oder Alkoxy mit jeweils 1 bis 6 Kohlenstoffatomen, Alkylthio mit 1 bis 6 Kohlenstoffatomen, Halogenalkyl, Halogenalkoxy oder Halogenalkylthio mit jeweils 1 bis 6 Kohlenstoffatomen und jeweils 1 bis 9 gleichen oder verschiedenen Halogenatomen, Nitro, Alkoxycarbonyl oder Alkylcarbonylamino mit 1 bis 4 Kohlenstoffatomen im Alkoxy oder Alkylteil steht,

$R^2$ für Halogen oder Alkyl mit 1 bis 6 Kohlenstoffatomen steht,

n für eine ganze Zahl 0, 1, 2, 3 oder 4 und

m für eine ganze Zahl 0, 1, 2 oder 3 stehen.

Besonders bevorzugt sind Verbindungen der Formel (I), in welcher

R für geradkettiges oder verzweigtes Alkyl mit 1 bis 4 Kohlenstoffatomen oder für geradkettiges oder verzweigtes Alkoxy mit 1 bis 4 Kohlenstoffatomen steht,

$R^1$ für Halogen, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy oder Halogenalkylthio mit jeweils 1 bis 4 Kohlenstoffatomen und jeweils 1 bis 5 gleichen oder verschiedenen Halogenatomen, Nitro, geradkettiges oder verzweigtes Alkoxycarbonyl mit 1 bis 3 Kohlenstoffatomen oder Alkylcarbonylamino mit 1 bis 3 Kohlenstoffatomen im Alkylteil steht,

2 für Halogen oder geradkettiges oder verzweigtes Alkyl mit 1 bis 4 Kohlenstoffatomen steht,

für eine ganze Zahl 0, 1, 2, 3 oder 4 und

für eine ganze Zahl 0, 1, 2 oder 3 stehen.

nsbesondere seien Verbindungen der Formel (I) genannt, n welchen

R für geradkettiges oder verzweigtes Alkyl oder Alkoxy mit jeweils 1 bis 4 Kohlenstoffatomen steht,

$R^1$ für Fluor, Chlor, geradkettiges oder verzweigtes Alkyl oder Alkoxy mit jeweils 1 bis 4 Kohlenstoffatomen, geradkettiges oder verzweigtes Alkylthio mit 1 bis 3 Kohlenstoffatomen, Halogenalkyl, Halogenalkoxy oder Halogenalkylthio mit jeweils 1 oder 2 Kohlenstoffatomen und jeweils 1 bis 4 gleichen oder verschiedenen Fluor- und Chloratomen, Nitro, Alkoxycarbonyl oder Alkylcarbonylamino mit jeweils 1 oder 2 Kohlenstoffatomen im Alkoxy- oder Alkylteil steht,

$R^2$ für Fluor, Chlor oder geradkettiges oder verzweigtes Alkyl mit 1 bis 4 Kohlenstoffatomen steht,

n für eine ganze Zahl 0, 1, 2, 3 oder 4 und

m für eine ganze Zahl 0, 1, 2 oder 3 stehen.

Ganz besonders bevorzugt seien die Verbindungen der Formel (I) genannt, in welchen

R für geradkettiges oder verzweigtes Alkyl oder Alkoxy mit jeweils 1 bis 4 Kohlenstoffatomen steht,

$R^1$ für Fluor, Chlor, Methyl, Ethyl, n-Propyl, iso-Propyl, Methoxy, Ethoxy, Methylthio, Trifluormethyl, Trifluormethoxy, Trifluormethylthio, Nitro, Methoxycarbonyl, Ethoxycarbonyl, Methylcarbonylamino oder Ethylcarbonylamino steht,

$R^2$ für Chlor, Methyl oder Ethyl steht,

für eine ganze Zahl 0, 1, 2 oder 3 und

m für eine ganze Zahl 0 oder 1 stehen.

Verwendet man 4-Methylphenylsulfonyl-2-pyridinaldoxim und Ameisensäureethylesterchlorid als Ausgangsstoffe, so läßt sich der Reaktionsablauf des erfindungsgemäßen Verfahrens durch das folgende Formelschema darstellen:

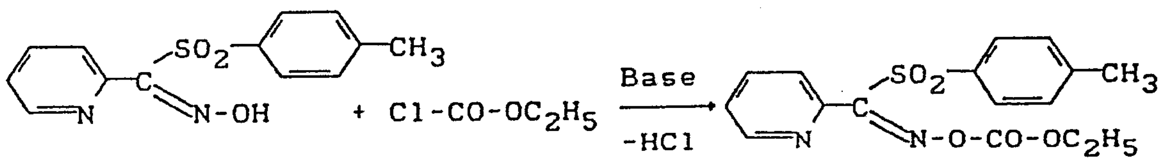


Die zur Durchführung des erfindungsgemäßen Verfahrens als Ausgangsstoffe benötigten Arylsulfonyl-pyridinaldoxime sind durch die Formel (II) definiert. Diese Stoffe sind neu und Gegenstand einer anderen noch nicht veröffentlichten Anmeldung. Die Verbindungen können aber nach Analogieverfahren z.B. hergestellt werden, indem man Pyridinaldoxime der Formel (IV)

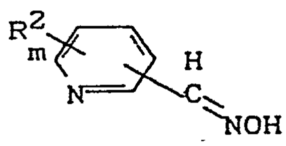

(IV)

in welcher

$R^2$ und m die oben angegebenen Bedeutungen haben,

mit Chlor, zu den entsprechenden α-Chlor-pyridinaldoximen umsetzt und diese mit Arylsulfinsäuren der Formel (V)

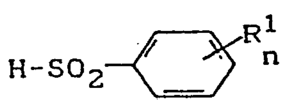

(V)

oder deren Alkaliverbindungen

in welcher

$R^1$ und n die oben angegebenen Bedeutungen haben,

gegebenenfalls in Gegenwart eines Lösungs- oder Verdünnungsmittels, wie z.B. Methanol, gegebenenfalls in Gegenwart einer Base, wie z.B. Triethylamin, bei gegebenenfalls erhöhten Temperaturen umsetzt.

Die Pyridinaldoxime der Formel (IV) und die Arylsulfinsäuren der Formel (V) sind bekannte Verbindungen der organischen Chemie.

Die weiterhin als Ausgangsstoffe benötigten Carbonylverbindungen sind durch die Formel (III) definiert. Es handelt sich um bekannte Verbindungen der organischen Chemie.

Das erfindungsgemäße Verfahren kann gegebenenfalls in Gegenwart eines Lösungs- oder Verdünnungsmittels durchgeführt werden. Als solche kommen prinzipiell alle inerten organischen Lösungsmittel infrage. Bevorzugt verwendet man Kohlenwasserstoffe, gegebenenfalls chlorierte, wie z.B. Benzol, Toluol, Xylol, Methylenchlorid, Chloroform, Tetrachlorkohlenstoff, Chlorbenzol und Dichlorbenzol, ferner Ether wie Diethyl- und Dibutylether, Glykoldimethylether und Diglykoldimethylether, Tetrahydrofuran und Dioxan, weiterhin Ketone wie Aceton, Methylethyl-, Methylisopropyl- und Methylisobutylketon, außerdem Ester, wie Essigsäure-methylester und -ethylester, ferner Nitrile wie z.B. Acetonitril und Propionitril, Benzonitril, Glutarsäuredinitril, darüber hinaus Amide wie z.B. Dimethylformamid.

Als Säurebindemittel kommen für das erfindungsgemäße Verfahren übliche anorganische oder organische Säurebinder infrage. Als solche seien genannt: z.B. tert. Amine wie Triethylamin, Pyridin, Triethylendiamin u.a.

Die Reaktionstemperatur des erfindungsgemäßen Verfahrens kann in einem größeren Temperaturbereich variiert werden. Im allgemeinen arbeitet man zwischen 0° C und 120° C, vorzugsweise zwischen 20° C und 70° C.

Die Reaktion wird normalerweise unter Normaldruck ausgeführt. Bei der Durchführung des erfindungsgemäßen Verfahrens legt man im allgemeinen die Verbindungen der Formel (II) in einem Lösungsmittel mit äquimolaren Mengen des Säurebinders vor und gibt die Carbonylverbindungen der Formel (III) vorzugsweise ebenfalls in äquimolaren Mengen zu. Die Aufarbeitung erfolgt nach allgemein üblichen Methoden.

Eine besondere Ausführungsform ist noch zu erwähnen. Wenn X die Bedeutung -O-COR hat, d.h. also Carbonsäureanhydride der Formel (III) eingesetzt werden, arbeitet man ohne Lösungsmittel mit einem hohen Überschuß des Anhydrids, das dann gleichzeitig als Ausgangsstoff und Lösungsmittel dient. Die Aufarbeitung erfolgt ebenfalls nach üblichen Verfahren.

Die erfindungsgemäßen Wirkstoffe weisen eine starke biologische Wirkung auf und können zur Bekämpfung von unerwünschten Schädlingen praktisch eingesetzt werden. Die Wirkstoffe sind für den Gebrauch als Schädlingsbekämpfungsmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;

Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;

Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielseise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe lassen sich mit besonders gutem Erfolg zur Bekämpfung von Gemüse- und Obstkrankheiten, verursacht z.B. durch Venturia inaequalis oder Botrytis cinerea, zur Bekämpfung von Reiskrankheiten, verursacht z.B. durch Pyricularia oryzae und zur Bekämpfung von Getreidekrankheiten, verursacht z.B. durch Puccinia recondita einsetzen.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder

Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine,

und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele

Beispiel 1

Vorprodukt:

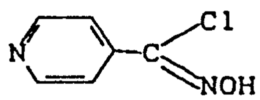 • HCl

40,6 g (0,333 m) Pyridin-4-aldoxim werden in 350 ml Methylenchlorid gelöst und es wird bei -15° C Chlor (getrocknet) bis zur Sättigung eingeleitet. Man läßt das Reaktionsgemisch über Nacht bis auf Raumtemperatur kommen und saugt dann ab. Das Reaktionsprodukt wird mit Methylenchlorid, dann mit Aceton gewaschen und mit Ether nachgespült. Man erhält 60 g (93,4 % der Theorie) der gewünschten Substanz mit einem Schmelzpunkt von 203° C (Zersetzung).

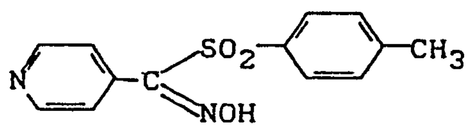

19,3 g (0,1 m) α-Chlor-pyridin-4-aldoxim-hydrochlorid werden in 250 ml Methanol gelöst und mit 18,7 g (0,105 m) 4-methylphenylsulfinsaurem Natrium versetzt. Zur Freisetzung des α-Chlorpyridin-4-aldoxims werden noch 14 ml (0,1 m) Triethylamin hinzugefügt. Die Reaktion verläuft schwach exotherm. Man hält das Reaktionsgemisch bei Zimmertemperatur und läßt bei dieser Temperatur über Nacht weiterrühren. Dann wird auf ca. 1 l Eiswasser gegossen, ausgerührt, abgesaugt, gewaschen und getrocknet. Das Reaktionsprodukt wird aus Isopropanol umkristallisiert. Man erhält 8,1 g (29 % der Theorie) der gewünschten Verbindung mit einem Schmelzpunkt von 138° C.

Endprodukt:

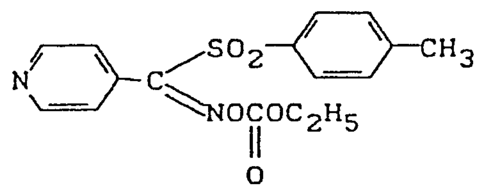

13,8 g (0,05 m) α-(4-Methylphenylsulfonyl)-pyridin-4-aldoxim werden in 150 ml Acetonitril gelöst und mit 7 ml (0,05 m) Triethylamin und 5,5 g (0,05 m) Chlorameisensäureethylester versetzt. Die Reaktion verläuft exotherm.

Das Reaktionsgemisch wird über Nacht bei Raumtemperatur gerührt, anschließend auf 750 ml Waser gegossen, ausgerührt, abgesaugt, gewaschen und getrocknet. Das Reaktionsprodukt wird aus Isopropanol umkristallisiert. Man erhält 7,4 g (43 % der Theorie) der gewünschten Verbindung mit einem Schmelzpunkt von 126° C.

Entsprechend Beispielen 1 und 9 können die Verbindungen der Formel (I) hergestellt werden:

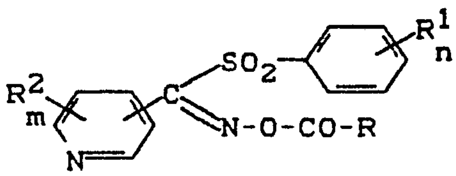

(I)

| Beispiel Nr. | R | $R^1$ | $R^2$ | n | m | Stellg. des Pyridinringes | Physikal. Daten [Schmelzpkt. °C] |
|---|---|---|---|---|---|---|---|
| 2 | $-CH_3$ | $4-CH_3$ | - | 1 | 0 | 4 | 54 |
| 3 | $-OC_2H_5$ | - | - | 0 | 0 | 4 | 108 |
| 4 | $-OC_2H_5$ | 4-Cl | - | 1 | 0 | 4 | 129 |
| 5 | $-OC_2H_5$ | 4-Cl | - | 1 | 0 | 3 | 43 |
| 6 | $-OC_2H_5$ | - | - | 0 | 0 | 3 | 148 |
| 7 | $-OC_2H_5$ | $4-CH_3$ | - | 1 | 0 | 3 | 133 |
| 8 | $-OCH_3$ | $4-CH_3$ | - | 1 | 0 | 2 | 138 |

Beispiel 9

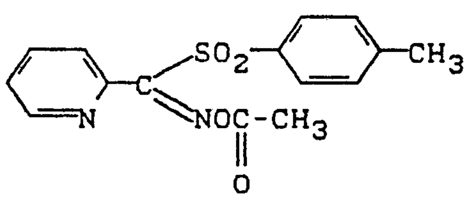

27,6 g (0,1 m) α-(4-Methyl-phenylsulfonyl)-pyridin-2-aldoxim werden mit 100 ml Acetanhydrid versetzt und über Nacht bei 50°C gerührt. Die Reaktionslösung wird filtriert, das Filtrat einrotiert und der verbleibende Rückstand aus Isopropanol umkristallisiert.
Ausbeute: 23,2 g = 73 % der Theorie; Schmelzpunkt: 88°C.

| Beispiel Nr. | R | $R^1$ | $R^2$ | n | m | Stellg. des Pyridinringes | Physikal. Daten [Schmelzpkt. °C] |
|---|---|---|---|---|---|---|---|
| 10 | $-OC_2H_5$ | $4-CH_3$ | $3-CH_3$ | 1 | 1 | 2 | 91 |
| 11 | $-OC_2H_5$ | - | $3-CH_3$ | 0 | 1 | 2 | 57 |
| 12 | $-OCH_3$ | 4-Cl | - | 1 | 0 | 2 | 75 |
| 13 | $-OC_2H_5$ | 4-Cl | - | 1 | 0 | 2 | 125 |
| 14 | $-OC_3H_7-i$ | 4-Cl | - | 1 | 0 | 2 | 82 |
| 15 | $-OC_3H_7-i$ | $4-CH_3$ | - | 1 | 0 | 3 | 133-34 |
| 16 | $-OCH_3$ | 4-Cl | - | 1 | 0 | 4 | 142 |
| 17 | $-OCH_3$ | - | - | 0 | 0 | 4 | 140 |
| 18 | $-OC_4H_9-i$ | $4-CH_3$ | - | 1 | 0 | 3 | 145 |
| 19 | $-OC_2H_5$ | $4-CH_3$ | - | 1 | 0 | 2 | 80 |
| 20 | $-OCH_3$ | 4-Cl | - | 1 | 0 | 3 | 147 |
| 21 | $-OC_3H_7-i$ | $4-CH_3$ | - | 1 | 0 | 2 | 98 |
| 22 | $-OCH_3$ | $4-CH_3$ | $3-CH_3$ | 1 | 1 | 2 | 119 |
| 23 | $-OCH_3$ | - | $3-CH_3$ | 0 | 1 | 2 | 101 |
| 24 | $-OCH_3$ | $4-CH_3$ | - | 1 | 0 | 3 | 161 |
| 25 | $-OCH_3$ | - | - | 0 | 0 | 3 | 94 |
| 26 | $-OC_4H_5-i$ | $4-CH_3$ | - | 1 | 0 | 2 | 84 |
| 27 | $-OC_4H_5-i$ | - | - | 0 | 0 | 3 | 92 |
| 28 | $-OCH_3$ | - | - | 0 | 0 | 2 | 113 |
| 29 | $-OC_2H_5$ | - | - | 0 | 0 | 2 | 92 |
| 30 | $-OC_4H_9-i$ | - | - | 0 | 0 | 2 | 53 |
| 31 | $-OCH_3$ | $4-CH_3$ | - | 1 | 0 | 4 | 150 |
| 32 | $-OC_4H_9-i$ | 4-Cl | - | 1 | 0 | 2 | 95 |
| 33 | $-OC_4H_9-i$ | 4-Cl | - | 1 | 0 | 3 | 145 |
| 34 | $-OC_4H_9-i$ | $4-CH_3$ | - | 1 | 0 | 4 | 125 |
| 35 | $-OC_4H_9-i$ | 4-Cl | - | 1 | 0 | 4 | 112 |
| 36 | $-OC_4H_9-i$ | - | - | 0 | 0 | 4 | 83 |
| 37 | $-OC_4H_9-i$ | $4-CH_3$ | $3-CH_3$ | 1 | 1 | 2 | 100 |
| 38 | $-OC_4H_9-i$ | - | $3-CH_3$ | 0 | 1 | 2 | 86 |

| Beispiel Nr. | R | $R^1$ | $R^2$ | n | m | Stellg. des Pyridinringes | Physikal. Daten [Schmelzpkt.°C] |
|---|---|---|---|---|---|---|---|
| 39 | $-OC_3H_7$-i | 4-$CH_3$ | 3-$CH_3$ | 1 | 1 | 2 | |
| 40 | $-OC_3H_7$ i | - | - | 0 | 0 | 2 | |
| 41 | $-OC_3H_7$-i | - | - | 0 | 0 | 2 | |
| 42 | $-OC_3H_7$-i | 4-Cl | - | 1 | 0 | 3 | |
| 43 | $-OC_3H_7$-i | - | - | 0 | 0 | 3 | |
| 44 | $-OC_3H_7$-i | 4-$CH_3$ | - | 1 | 0 | 4 | |
| 45 | $-OC_3H_7$-i | 4-Cl | - | 1 | 0 | 4 | |
| 46 | $-OC_3H_7$-i | - | - | 0 | 0 | 4 | |
| 47 | $-OC_3H_7$-i | 4-$CH_3$ | 3-$CH_3$ | 1 | 1 | 2 | |
| 48 | $-OC_3H_7$-i | - | 3-$CH_3$ | 0 | 1 | 2 | |

Beispiel

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Die erfindungsgemäßen Verbindungen zeigen eine gute Wirksamkeit bei einer Wirkstoffkonzentration von 0,025 %.

T a b e l l e

Pyricularia-Test (Reis) / protektiv

| Wirkstoffe | Wirkstoffkonzentration in % | Krankheitsbefall in % der unbehandelten Kontrolle |
|---|---|---|
| 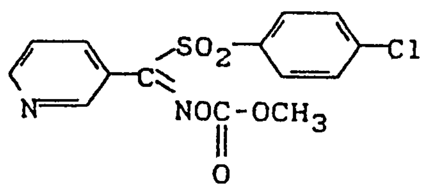 | 0,025 | 22 |
| 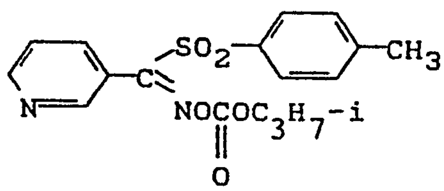 | 0,025 | 11 |
| 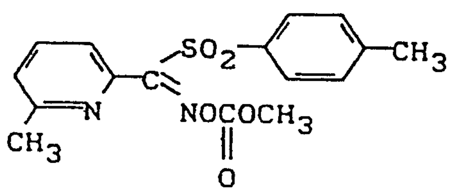 | 0,025 | 0 |
| 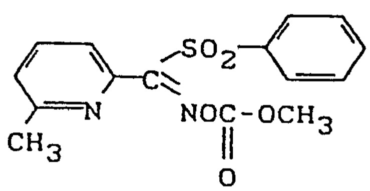 | 0,025 | 0 |

Beispiel

Phytophthora-Test (Tomate) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Die erfindungsgemäßen Verbindungen zeigen eine gute Wirksamkeit.

Beispiel

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20° C und 100 % relativer Luftfeuchtigkeit in einer Inkubationskabine.

Die Pflanzen werden dann im Gewächshaus bei 20° C und einer relativen Luftfeuchtigkeit von ca. 70 % aufgestellt.

12 Tage nach der Inokulation erfolgt die Auswertung.

Beispiel

Botrytis-Test (Bohne)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Die erfindungsgemäßen Verbindungen zeigen eine gute Wirksamkeit.

Patentansprüche

1. Arylsulfonyl-pyridinaldoxim-Derivate der allgemeinen Formel (I)

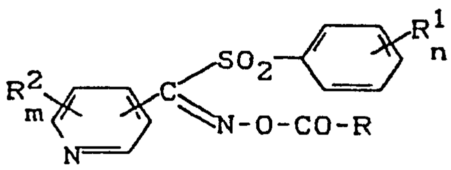

(I)

in welcher

R für Alkyl oder Alkoxy st ht,

$R^1$ für Halogen, Alkyl, Alkoxy, Alkylthio, Halogenalkyl, Halogenalkoxy, Halogenalkylthio, Nitro, Alkoxycarbonyl oder Alkylcarbonylamino steht,

$R^2$ für Halogen oder Alkyl steht,

n für eine ganze Zahl 0, 1, 2, 3, 4 oder 5 und

m für eine ganze Zahl 0, 1, 2, 3 oder 4 stehen.

2. Arylsulfonyl-pyridinaldoxim-Derivate gemäß Anspruch 1, wobei in Formel (I)

R für geradkettiges oder verzweigtes Alkyl mit 1 bis 6 Kohlenstoffatomen oder Alkoxy mit 1 bis 6 Kohlenstoffatomen steht,

$R^1$ für Halogen, Alkyl oder Alkoxy mit jeweils 1 bis 6 Kohlenstoffatomen, Alkylthio mit 1 bis 6 Kohlenstoffatomen, Halogenalkyl, Halogenalkoxy oder Halogenalkylthio mit jeweils 1 bis 6 Kohlenstoffatomen und jeweils 1 bis 9 gleichen oder verschiedenen Halogenatomen, Nitro, Alkoxycarbonyl oder Alkylcarbonylamino mit 1 bis 4 Kohlenstoffatomen im Alkoxy- oder Alkylteil steht,

$R^2$ für Halogen oder Alkyl mit 1 bis 6 Kohlenstoffatomen steht,

r für eine ganze Zahl 0, 1, 2, 3 oder 4 und

m für eine ganze Zahl 0, 1, 2 oder 3 stehen.

3. Arylsulfonyl-pyridinaldoxim-Derivate gemäß Anspruch 1, wobei in der Formel (I)

R für geradkettiges oder verzweigtes Alkyl mit 1 bis 4 Kohlenstoffatomen oder für geradkettiges oder verzweigtes Alkoxy mit 1 bis 4 Kohlenstoffatomen steht,

$R^1$ für Halogen, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, Halogenalkyl mit 1 bis 4 Kohlenstoffatomen und 1 bis 5 gleichen oder verschiedenen Halogenatomen,

Halogenalkoxy oder Halogenalkylthio mit jeweils 1 bis 4 Kohlenstoffatomen und jeweils 1 bis 5 gleichen oder verschiedenen Halogenatomen, Nitro, geradkettiges oder verzweigtes Alkoxycarbonyl mit 1 bis 3 Kohlenstoffatomen oder Alkylcarbonylamino mit 1 bis 3 Kohlenstoffatomen im Alkylteil steht,

$R^2$ für Halogen oder geradkettiges oder verzweigtes Alkyl mit 1 bis 4 Kohlenstoffatomen steht,

n für eine ganze Zahl 0, 1, 2, 3 oder 4 und

m für eine ganze Zahl 0, 1, 2 oder 3 stehen.

4. Arylsulfonyl-pyridinaldoxim-Derivate gemäß Anspruch 1, wobei in der Formel (I)

R für geradkettiges oder verzweigtes Alkyl oder Alkoxy mit jeweils 1 bis 4 Kohlenstoffatomen steht,

$R^1$ für Fluor, Chlor, geradkettiges oder verzweigtes Alkyl oder Alkoxy mit jeweils 1 bis 4 Kohlenstoffatomen, geradkettiges oder verzweigtes Alkylthio mit 1 bis 3 Kohlenstoffatomen, Halogenalkyl, Halogenalkoxy oder Halogenalkylthio mit jeweils 1 oder 2 Kohlenstoffatomen und jeweils 1 bis 4 gleichen oder verschiedenen Fluor- und Chloratomen, Nitro, Alkoxycarbonyl oder Alkylcarbonylamino mit jeweils 1 oder 2 Kohlenstoffatomen im Alkoxy- oder Alkylteil steht,

$R^2$ für Fluor, Chlor oder geradkettiges oder verzweigtes Alkyl mit 1 bis 4 Kohlenstoffatomen steht,

n für eine ganze Zahl 0, 1, 2, 3 oder 4 und

m für eine ganze Zahl 0, 1, 2 oder 3 stehen.

5. Arylsulfonyl-pyridinaldoxim-Derivate gemäß Anspruch 1, wobei in der Formel (I)

R für geradkettiges oder verzweigtes Alkyl oder Alkoxy mit jeweils 1 bis 4 Kohlenstoffatomen steht,

$R^1$ für Fluor, Chlor, Methyl, Ethyl, n-Propyl, iso-Propyl, Methoxy, Ethoxy, Methylthio, Trifluormethyl, Trifluormethoxy, Trifluormethylthio, Nitro, Methoxycarbonyl, Ethoxycarbonyl, Methylcarbonylamino oder Ethylcarbonylamino steht,

$R^2$ für Chlor, Methyl oder Ethyl steht,

n für eine ganze Zahl 0, 1, 2 oder 3 und

m für eine ganze Zahl 0 oder 1 stehen.

6. Verfahren zur Herstellung von Arylsulfonyl-pyridinaldoxim-Derivaten der allgemeinen Formel (I)

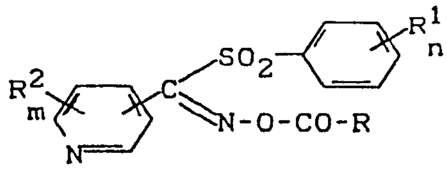

(I)

in welcher

R für Alkyl oder Alkoxy steht,

$R^1$ für Halogen, Alkyl, Alkoxy, Alkylthio, Halogenalkyl, Halogenalkoxy, Halogenalkylthio, Nitro, Alkoxycarbonyl oder Alkylcarbonylamino steht,

$R^2$ für Halogen oder Alkyl steht,

n für eine ganze Zahl 0, 1, 2, 3, 4 oder 5 und

m für eine ganze Zahl 0, 1, 2, 3 oder 4 stehen,

dadurch gekennzeichnet, daß man Arylsulfonyl-pyridinaldoxime der allgemeinen Formel (II)

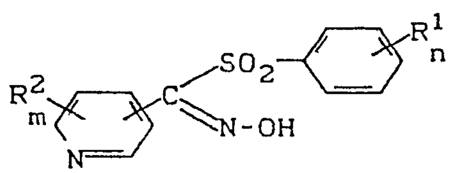

(II)

in welcher

$R^1$, $R^2$, n und m die oben angegebenen Bedeutungen haben, mit Carbonylverbindungen der allgemeinen Formel (III)

$$X - CO - R \qquad (III)$$

in welcher

R die oben angegebenen Bedeutungen hat und

X für ein Halogenatom, vorzugsweise Chlor, oder für den Rest -O-COR steht,

wobei R die oben angegebenen Bedeutungen hat, gegebenenfalls in Gegenwart eines Säurebindemittels umsetzt.

7. Schädlingsbekämpfungsmittel, gekennzeichnet durch einen Gehalt an mindestens einem Arylsulfonyl-pyridinaldoxim-Derivat der Formel (I) gemäß den Ansprüchen 1 bis 6.

8. Verfahren von Arylsulfonyl-pyridinaldoxim-Derivaten der Formel (I) nach den Ansprüchen 1 bis 6 zur Bekämpfung von Schädlingen.

9. Verfahren zur Bekämpfung von Schädlingen, dadurch gekennzeichnet, daß man Arylsulfonyl-pyridinaldoxim-Derivate der Formel (I) nach den Ansprüchen 1 bis 6 auf Schädlinge und/oder ihren Lebensraum einwirken läßt.

10. Verfahren zur Herstellung von Schädlingsbekämpfungsmitteln, dadurch gekennzeichnet, daß man Arylsulfonyl-pyridinaldoxim-Derivate der Formel (I) nach den Ansprüchen 1 bis 6 mit Streckmitteln und/oder oberflächenaktiven Mitteln vermischt.

Europäisches Patentamt

# EUROPÄISCHER RECHERCHENBERICHT

Nummer der Anmeldung 0236897

EP 87102912.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| D,A | CH - A - 423 350 (SHELL)<br>* Ansprüche 1,13 *<br>-- | 1,7 | C 07 D 213/78<br>A 01 N 43/40<br>A 01 N 47/06 |
| A | EP - A2 - 0 136 640 (NIHON)<br>* Zusammenfassung; Ansprüche 1,4,5,8 *<br>-- | 1,6,7,10 | |
| A | CH - A5 - 651 552 (HOFFMANN-LA ROCHE)<br>* Zusammenfassung; Anspruch 1 *<br>---- | 1,7 | |

RECHERCHIERTE SACHGEBIETE (Int Cl 4)

C 07 D 213/00

Der vorliegende Recherchenbericht wurde für alle Patentansprüche erstellt.

| Recherchenort | Abschlußdatum der Recherche | Prüfer |
|---|---|---|
| WIEN | 16-06-1987 | HOCHHAUSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82